# EUROPÄISCHE PATENTSCHRIFT

(11) **EP 2 588 295 B1**
(45) Veröffentlichungstag und Bekanntmachung des Hinweises auf die Patenterteilung: **30.05.2018**
(21) Anmeldenummer: 11755242.2
(22) Anmeldetag: 27.06.2011
(51) Int. Cl.: B29C 49/42, B29C 49/46, B29C 49/64, A61L 2/20, B29C 49/06, B29C 49/12, B29C 49/16, B29C 49/36

(54) **VERFAHREN UND VORRICHTUNG ZUM STERILISIEREN VON VORFORMLINGEN**
METHOD AND DEVICE FOR STERILIZING PREFORMS
PROCÉDÉ ET DISPOSITIF DE STÉRILISATION DE PARAISONS

(30) Priorität: 01.07.2010 DE 102010026166
(43) Veröffentlichungstag der Anmeldung: 08.05.2013
(73) Patentinhaber: KHS Corpoplast GmbH, 22145 Hamburg (DE); KHS GmbH, 44143 Dortmund (DE)
(72) Erfinder: HEROLD, Thomas, 22926 Ahrensburg (DE); RIEGER, Harald, 22303 Hamburg (DE); KLATT, Dieter, 22147 Hamburg (DE); HAESENDONCKX, Frank, 22047 Hamburg (DE)
(74) Vertreter: Hausfeld, Norbert
(86) Internationale Anmeldenummer: PCT/DE2011/001373
(87) Internationale Veröffentlichungsnummer: WO 2012/000486

(56) Entgegenhaltungen:
- EP-A1- 1 941 913
- EP-B1- 1 896 329
- WO-A1-2010/020530
- DE-A1-102008 056 346
- JP-A- 3 290 226

## Beschreibung

Die Erfindung betrifft ein Verfahren zum Sterilisieren von Vorformlingen aus einem thermoplastischen Material, die zur Herstellung von blasgeformten Behältern vorgesehen sind sowie bei dem ein Sterilisationsmittel in den Bereich des Vorformlings zugeführt wird, sowie bei dem die Vorformlinge vor ihrer Blasverformung erwärmt werden. Die Erwärmung erfolgt in einer Heizstrecke, in der entlang einer Transportrichtung der Vorformlinge Heizstrahler einseitig angeordnet sind.

Darüber hinaus betrifft die Erfindung eine Vorrichtung zum Blasformen von mindestens bereichsweise sterilen Behältern durch Verformung eines Vorformlings, die eine Zuführeinrichtung zur Beaufschlagung mindestens eines Teiles des Vorformlings mit einem Sterilisationsmittel aufweist.

Schließlich betrifft die Erfindung eine Vorrichtung zum Sterilisieren von Vorformlingen aus einem thermoplastischen Material, die zur Herstellung von blasgeformten Behältern vorgesehen sind. Ebenfalls ist von der Erfindung eine Vorrichtung zur Herstellung von mindestens bereichsweise sterilen Behältern aus einem thermoplastischen Material umfaßt.

Die Erfindung betrifft auch eine Vorrichtung zur Blasformung von Behältern, die mindestens eine auf einer Tragstruktur angeordnete Blasstation zur Umformung von thermoplastischen Vorformlingen in die Behälter aufweist.

Eine Herstellung von sterilen blasgeformten Behältern erfolgt typischerweise derart, daß diese Behälter nach ihrer Blasformung und vor einer Befüllung unter Verwendung von Wasserstoffperoxid oder anderen Chemikalien sterilisiert werden. Ebenfalls ist es bereits bekannt, die bei der Blasformung der Behälter als Ausgangsprodukt verwendeten Vorformlinge zu sterilisieren, insbesondere den Bereich der inneren Oberfläche dieser Vorformlinge.

Bei einer Behälterformung durch Blasdruckeinwirkung werden Vorformlinge aus einem thermoplastischen Material, beispielsweise Vorformlinge aus PET (Polyethylenterephtalat), innerhalb einer Blasmaschine unterschiedlichen Bearbeitungsstationen zugeführt. Typischerweise weist eine derartige Blasmaschine eine Heizeinrichtung sowie eine Blaseinrichtung auf, in deren Bereich der zuvor temperierte Vorformling durch biaxiale Orientierung zu einem Behälter expandiert wird. Die Expansion erfolgt mit Hilfe von Druckluft, die in den zu expandierenden Vorformling eingeleitet wird. Der Verfahrenstechnische Ablauf bei einer derartigen Expansion des Vorformlings wird in der DE-OS 43 40 291 erläutert.

Der grundsätzliche Aufbau einer Blasstation zur Behälterformung wird in der DE-OS 42 12 583 beschrieben. Möglichkeiten zur Temperierung der Vorformlinge werden in der DE - OS 23 52 926 erläutert.

Innerhalb der Vorrichtung zur Blasformung können die Vorformlinge sowie die geblasenen Behälter mit Hilfe unterschiedlicher Handhabungseinrichtungen transportiert werden. Bewährt hat sich insbesondere die Verwendung von Transportdornen, auf die die Vorformlinge aufgesteckt werden. Die Vorformlinge können aber auch mit anderen Trageinrichtungen gehandhabt werden. Die Verwendung von Greifzangen zur Handhabung von Vorformlingen und die Verwendung von Spreizdornen, die zur Halterung in einen Mündungsbereich des Vorformlings einführbar sind, gehören ebenfalls zu den verfügbaren Konstruktionen.

Eine Handhabung von Behältern unter Verwendung von übergaberädern wird beispielsweise in der DE-OS 199 06 438 bei einer Anordnung des Übergaberades zwischen einem Blasrad und einer Ausgabestrecke beschrieben.

Die bereits erläuterte Handhabung der Vorformlinge erfolgt zum einen bei den sogenannten Zweistufenverfahren, bei denen die Vorformlinge zunächst in einem Spritzgußverfahren hergestellt, anschließend zwischengelagert und erst später hinsichtlich ihrer Temperatur konditioniert und zu einem Behälter aufgeblasen werden. Zum anderen erfolgt eine Anwendung bei den sogenannten Einstufenverfahren, bei denen die Vorformlinge unmittelbar nach ihrer spritzgußtechnischen Herstellung und einer ausreichenden Verfestigung geeignet temperiert und anschließend aufgeblasen werden.

Im Hinblick auf die verwendeten Blasstationen sind unterschiedliche Ausführungsformen bekannt. Bei Blasstationen, die auf rotierenden Transporträdern angeordnet sind, ist eine buchartige Aufklappbarkeit der Formträger häufig anzutreffen. Es ist aber auch möglich, relativ zueinander verschiebliche oder andersartig geführte Formträger einzusetzen. Bei ortsfesten Blasstationen, die insbesondere dafür geeignet sind, mehrere Kavitäten zur Behälterformung aufzunehmen, werden typischerweise parallel zueinander angeordnete Platten als Formträger verwendet.

Hinsichtlich der Sterilisierung von Vorformlingen sind aus dem Stand der Technik bereits unterschiedliche Verfahren und Vorrichtungen bekannt, die jedoch alle verfahrensspezifische Nachteile aufweisen, die einer zuverlässigen Sterilisierung der Vorformlinge bei gleichzeitig hohen Durchsatzraten entgegenstehen.

In der EP-A 1 086 019 wird beispielsweise die Sterilisierung von heißen Vorformlingen mit einem heißen gasförmigen Sterilisationsmittel beschrieben. Es werden hintereinander angeordnete separate Behandlungsstationen verwendet, nämlich ein erstes Heizmodul, ein Sterilisiermodul sowie ein zweites Heizmodul. Nachteilig ist hierbei das Temperaturverhalten des Vorformlings während des Sterilisiervorganges sowie das unkontrollierte Austreten des Sterilisationsmittels aus dem Vorformling innerhalb der Heizung.

In der EP-A 1 896 245 wird ein Verfahren beschrieben, bei dem vor der Heizung ein gasförmiges Sterilisationsmittel in einen kalten Vorformling eingeleitet wird und hier kondensiert. Problematisch ist hier die Sicherstellung einer vollständigen Kondensatbildung auf der gesamten Innenfläche des Vorformlings, da das einströmende heiße Sterilisationsmittel die Innenwandtemperatur des Vorformlings erhöht. Darüber hinaus tritt auch hier das Sterilisationsmittel nach seiner Verdampfung im Bereich der Heizung unkontrolliert innerhalb der Heizung aus dem Vorformling aus.

In der EP-A 2 138 298 wird eine Vorrichtung beschrieben, bei der vorsorglich sowohl vor dem verwendeten Blasmodul als auch hinter dem verwendeten Blasmodul Sterilisiereinrichtungen angeordnet sind. Hieraus resultiert ein sehr großer maschinenbaulicher Aufwand.

In der WO 2010/020530 A1 wird die Anordnung einer Sterilisiereinrichtung zwischen einer Heizung und dem Blasmodul beschrieben. Bei diesem Verfahren ist die Eintragsmenge von Sterilisationsmittel in den Bereich des Blasmoduls nur schwer vorhersehbar. Darüber hinaus ist die Austrittsmenge an Sterilisationsmittel in die Umgebung nicht kontrollierbar und eine entsprechende Kontamination nicht ausgeschlossen.

In der DE 10 2008 056 346 A1 werden Verfahren und Vorrichtungen gezeigt, bei denen die Temperierung der Vorformlinge auf einem Heizrad mit rotierenden Heizstationen durch Mikrowellenbestrahlung erfolgt. Eine Sterilisierung der Vorformlinge erfolgt durch Einsprüchen eines Sterilisationsmediums, das mit der Mikrowellenstrahlung wechselwirkt.

Die JP 3 290 226 A zeigt das Sterilisieren von Vorformlingen durch Eintauchen in ein Sterilisierbad. Nachfolgend wird der sterilisierte Vorformling auf die Blasformungstemperatur gebracht und im Anschluss in einen Behälter umgeformt.

Die EP 1 941 913 A1 zeigt die Sterilisierung von Vorformlingen mittels Einsprühen eines chemischen Sterilisationsmittels von oben in nach oben offene Vorformlinge. Die sterilisierten Vorformlinge werden anschließend gewendet und in einer Heizeinrichtung auf Blasformungstemperatur gebracht. Abschließend werden die Vorformlinge in Behälter umgeformt.

Die EP 1 896 329 B1 offenbart zusätzlich zum oben angegebenen Inhalt der EP 1 941 913 A1, dass zunächst ein Aufheizen der Vorformlinge in einer Heizeinrichtung erfolgen kann und dass danach ein Sterilisationsmittel von oben in die nach oben offenen Vorformlinge gesprüht wird. Im Anschluss erfolgt die Umformung der Vorformlinge in Behälter.

Aufgabe der vorliegenden Erfindung ist es, ein Verfahren der einleitend genannten Art derart zu verbessern, daß in einfacher Weise eine zuverlässige Sterilisation durchgeführt werden kann.

Diese Aufgabe wird erfindungsgemäß dadurch gelöst, daß das Sterilisieren mindestens zeitweise während des Erwärmens der Vorformlinge in der Heizstrecke durchgeführt wird. Zur Erreichung einer Aktivierung des Sterilisationsmittels ist weiterhin vorgesehen, daß die Temperatur des Vorformlings im Bereich einer inneren Oberfläche während der Durchführung der Sterilisierung mindestens 80 °C beträgt.

Weitere Aufgabe der vorliegenden Erfindung ist es, eine Vorrichtung der einleitend genannten Art derart zu konstruieren, daß eine effektive Sterilisation mit geringem Aufwand durchführbar ist.

Diese Aufgabe wird erfindungsgemäß dadurch gelöst, daß die Zuführeinrichtung im Bereich einer Heizstrecke zur Temperierung der Vorformlinge angeordnet ist. Die Erwärmung der Vorformlinge erfolgt in der Heizstrecke, in der entlang einer Transportrichtung der Vorformlinge Heizstrahler einseitig angeordnet sind. Der Vorformling ist im Bereich mindestens einer Transportposition innerhalb der Heizstrecke sowohl von einem Heizelement mit Wärmeenergie als auch von der Zuführeinrichtung mit dem Sterilisationsmittel beaufschlagbar, wobei sowohl die in Transportrichtung der Vorformlinge vor der Zuführeinrichtung als auch die im Bereich der Zuführeinrichtung angeordneten Heizelemente dazu ausgebildet sind, den Vorformling mindestens im Bereich seiner inneren Oberfläche mit einer Temperatur von mindestens 80°C zu erwärmen.

Schließlich besteht eine Aufgabe der vorliegenden Erfindung darin, eine Vorrichtung zur Blasformung von Behältern der einleitend genannten Art derart zu konstruieren, daß die Herstellung von sterilen Behältern unterstützt wird.

Diese Aufgabe wird erfindungsgemäß dadurch gelöst, daß im Bereich einer Heizstrecke Heizelemente zur Temperierung der Vorformlinge angeordnet ist und daß der Vorformling im Bereich mindestens einer Transportposition innerhalb der Heizstrecke sowohl von einem Heizelement mit Wärmeenergie als auch von der Zuführeinrichtung mit dem Sterilisationsmittel beaufschlagbar ist.

Durch das gleichzeitige Beheizen und Sterilisieren der Vorformlinge wird zum einen eine kompakte Bauform und eine kurze Prozeßzeit unterstützt. Darüber hinaus sorgt die Beheizung der Vorformlinge während des Sterilisierens für eine zusätzliche Aktivierung des Sterilisationsmittels und somit für eine Beschleunigung des Sterilisiervorganges. Schließlich sorgt die Beheizung gleichzeitig zur Durchführung des Sterilisationsvorganges auch dafür, daß Reste des Sterilisationsmittels schnell und rückstandsarm aus dem Bereich des Vorformlings entfernt werden können.

Als besonders vorteilhaft für eine Aktivierung erweist es sich, daß die Temperatur in einem Bereich von 100 °C bis 130 °C liegt.

Die Erreichung einer erforderlichen Sterilisationstemperatur, die Beibehaltung dieser Temperatur während des Sterilisierens und ein der Sterilisierung nachfolgendes Trocknen werden dadurch unterstützt, daß der Vorformling vor dem Sterilisieren, während des Sterilisierens sowie nach dem Sterilisieren erwärmt wird.

Zur Vermeidung unerwünschter Verformungen der Vorformlinge wird vorgeschlagen, daß ein Mündungsabschnitt des Vorformlings mindestens während eines Teiles des Erwärmungsvorganges gekühlt wird.

Ein Austreten von Sterilisationsmittel im Bereich der Heizung kann dadurch vermieden werden, daß mindestens während eines Teiles des Zeitraumes des Sterilisierens das Sterilisationsmittel sowohl in einen Innenraum des Vorformlings eingeleitet als auch aus diesem abgeleitet wird.

Insbesondere erweist es sich dabei als vorteilhaft, daß der Innenraum des Vorformlings gegenüber einer Umgebung des Vorformlings verschlossen wird.

In den Zeichnungen sind Ausführungsbeispiele der Erfindung schematisch dargestellt. Es zeigen:
- Fig. 1: Eine perspektivische Darstellung einer Blasstation zur Herstellung von Behältern aus Vorformlingen,
- Fig. 2: einen Längsschnitt durch eine Blasform, in der ein Vorformling gereckt und expandiert wird,
- Fig. 3: eine Skizze zur Veranschaulichung eines grundsätzlichen Aufbaus einer Vorrichtung zur Blas-formung von Behältern,
- Fig. 4: eine modifizierte Heizstrecke mit vergrößerter Heizkapazität,
- Fig. 5: eine schematische Darstellung eines Heizmoduls einer Blasmaschine, bei der im Bereich des Heizmoduls eine Sterilisiereinrichtung angeordnet ist,
- Fig. 6: eine gegenüber Fig. 5 abgewandelte Ausführungsform und
- Fig. 7: einen Längsschnitt durch einen Applikator für das Sterilisationsmittel, der in einen Mündungsbereich des Vorformlings eingeführt im Bereich des Heizmoduls positioniert ist.

Vor einer Erläuterung des Detailaufbaus der Vorrichtung zum Sterilisieren der Vorformlinge (1) durch Anwendung eines Sterilisationsmittels sowie vor einer Erläuterung eines konkreten Einbaues einer entsprechenden Vorrichtung in eine Blasmaschine soll nachfolgend zunächst der grundsätzliche Aufbau einer Blasmaschine beschrieben werden.

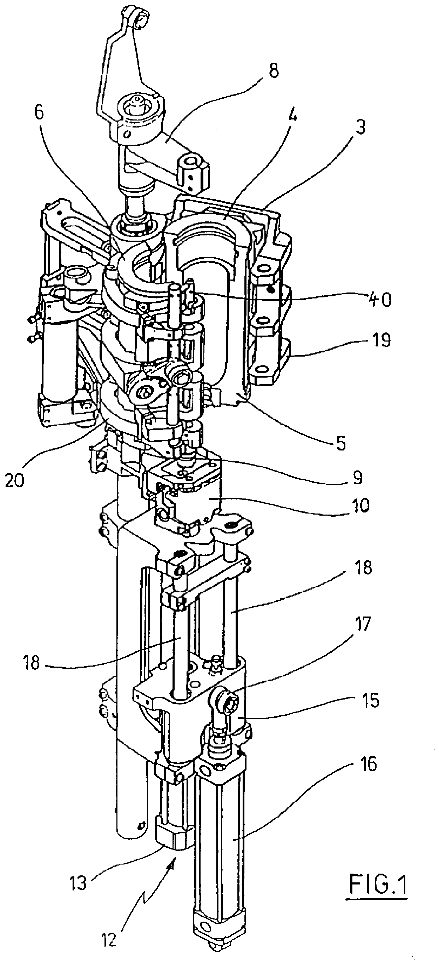

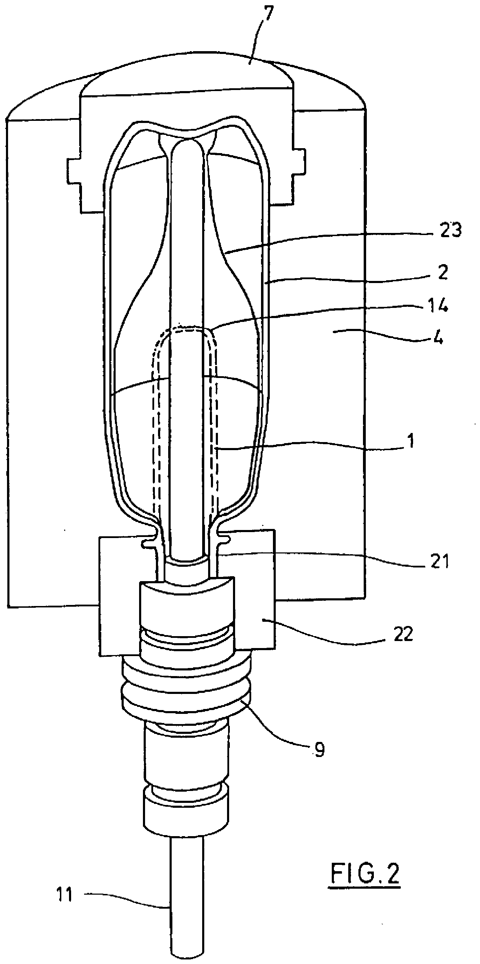

Der prinzipielle Aufbau einer Vorrichtung zur Umformung von Vorformlingen (1) in Behälter (2) ist in Fig. 1 und in Fig. 2 dargestellt.

Die Vorrichtung zur Formung des Behälters (2) besteht im wesentlichen aus einer Blasstation (3), die mit einer Blas-form (4) versehen ist, in die ein Vorformling (1) einsetz-bar ist. Der vorformling (1) kann ein spritzgegossenes Teil aus Polyethylenterephthalat sein. Zur Ermöglichung eines Einsetzens des Vorformlings (1) in die Blasform (4) und zur Ermöglichung eines Herausnehmens des fertigen Behälters (2) besteht die Blasform (4) aus Formhälften (5, 6) und einem Bodenteil (7), das von einer Hubvorrichtung (8) positio-nierbar ist. Der Vorformling (1) kann im Bereich der Blas-station (3) von einem Transportdorn (9) gehalten sein, der gemeinsam mit dem Vorformling (1) eine Mehrzahl von Behandlungsstationen innerhalb der Vorrichtung durchläuft. Es ist aber auch möglich, den Vorformling (1) beispiels-weise über Zangen oder andere Handhabungsmittel direkt in die Blasform (4) einzusetzen.

Zur Ermöglichung einer Druckluftzuleitung ist unterhalb des Transportdornes (9) ein Anschlußkolben (10) angeordnet, der dem vorformling (1) Druckluft zuführt und gleichzeitig eine Abdichtung relativ zum Transportdorn (9) vornimmt. Bei einer abgewandelten Konstruktion ist es grundsätzlich aber auch denkbar, feste Druckluftzuleitungen zu verwenden.

Eine Reckung des Vorformlings (1) erfolgt mit Hilfe einer Reckstange (11), die von einem Zylinder (12) positioniert wird. Grundsätzlich ist es aber auch denkbar, eine mecha-nische Positionierung der Reckstange (11) über Kurven-segmente durchzuführen, die von Abgriffrollen beaufschlagt sind. Die Verwendung von Kurvensegmenten ist insbesondere dann zweckmäßig, wenn eine Mehrzahl von Blasstationen (3) auf einem rotierenden Blasrad angeordnet sind. Eine Ver-wendung von Zylindern (12) ist zweckmäßig, wenn ortsfest angeordnete Blasstationen (3) vorgesehen sind.

Bei der in Fig. 1 dargestellten Ausführungsform ist das Recksystem derart ausgebildet, daß eine Tandem-Anordnung von zwei Zylindern (12) bereitgestellt ist. Von einem Primärzylinder (13) wird die Reckstange (11) zunächst vor Beginn des eigentlichen Reckvorganges bis in den Bereich eines Bodens (14) des Vorformlings (1) gefahren. Während des eigentlichen Reckvorganges wird der Primärzylinder (13) mit ausgefahrener Reckstange gemeinsam mit einem den Primärzylinder (13) tragenden Schlitten (15) von einem Sekundärzylinder (16) oder über eine Kurvensteuerung positioniert. Insbesondere ist daran gedacht, den Sekundär-zylinder (16) derart kurvengesteuert einzusetzen, daß von einer Führungsrolle (17), die während der Durchführung des Reckvorganges an einer Kurvenbahn entlang gleitet, eine aktuelle Reckposition vorgegeben wird. Die Führungsrolle (17) wird vom Sekundärzylinder (16) gegen die Führungsbahn gedrückt. Der Schlitten (15) gleitet entlang von zwei Führungselementen (18) .

Nach einem Schließen der im Bereich von Trägern (19, 20) angeordneten Formhälften (5, 6) erfolgt eine Verriegelung der Träger (19, 20) relativ zueinander mit Hilfe einer Verriegelungseinrichtung (40).

Zur Anpassung an unterschiedliche Formen eines Mündungsabschnittes (21) des vorformlings (1) ist gemäß Fig. 2 die Verwendung separater Gewindeeinsätze (22) im Bereich der Blasform (4) vorgesehen.

Fig. 2 zeigt zusätzlich zum geblasenen Behälter (2) auch gestrichelt eingezeichnet den Vorformling (1) und schematisch eine sich entwickelnde Behälterblase (23).

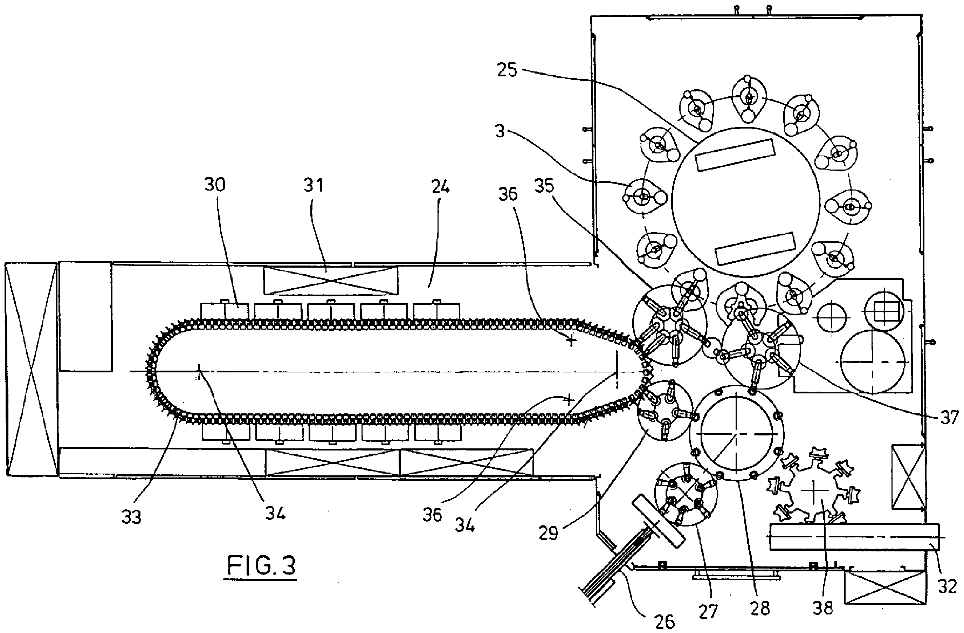

Fig. 3 zeigt den grundsätzlichen Aufbau einer Blasmaschine, die mit einer Heizstrecke (24) sowie einem rotierenden Blasrad (25) versehen ist. Ausgehend von einer Vorformlingseingabe (26) werden die Vorformlinge (1) von Übergaberädern (27, 28, 29) in den Bereich der Heizstrecke (24) transportiert. Entlang der Heizstrecke (24) sind Heizstrahler (30) sowie Gebläse (31) angeordnet, um die Vorformlinge (1) zu temperieren. Nach einer ausreichenden Temperierung der Vorformlinge (1) werden diese an das Blasrad (25) übergeben, in dessen Bereich die Blasstationen (3) angeordnet sind. Die fertig geblasenen Behälter (2) werden von weiteren Übergaberädern einer Ausgabestrecke (32) zugeführt.

Um einen Vorformling (1) derart in einen Behälter (2) umformen zu können, daß der Behälter (2) Materialeigenschaften aufweist, die eine lange Verwendungsfähigkeit von innerhalb des Behälters (2) abgefüllten Lebensmitteln, insbesondere von Getränken, gewährleisten, müssen spezielle Verfahrensschritte bei der Beheizung und Orientierung der Vorformlinge (1) eingehalten werden. Darüber hinaus können vorteilhafte Wirkungen durch Einhaltung spezieller Dimensionierungsvorschriften erzielt werden.

Als thermoplastisches Material können unterschiedliche Kunststoffe verwendet werden. Einsatzfähig sind bei-spielsweise PET, PEN oder PP.

Die Expansion des vorformlings (1) während des Orientierungsvorganges erfolgt durch Druckluftzuführung. Die Druckluftzuführung ist in eine Vorblasphase, in der Gas, zum Beispiel Preßluft, mit einem niedrigen Druckniveau zugeführt wird und in eine sich anschließende Hauptblasphase unterteilt, in der Gas mit einem höheren Druckniveau zugeführt wird. Während der Vorblasphase wird typischerweise Druckluft mit einem Druck im Intervall von 10 bar bis 25 bar verwendet und während der Hauptblasphase wird Druckluft mit einem Druck im Intervall von 25 bar bis 40 bar zugeführt.

Aus Fig. 3 ist ebenfalls erkennbar, daß bei der dargestellten Ausführungsform die Heizstrecke (24) aus einer Vielzahl umlaufender Transportelemente (33) ausgebildet ist, die kettenartig aneinandergereiht und entlang von Umlenkrädern (34) geführt sind. Insbesondere ist daran gedacht, durch die kettenartige Anordnung eine im wesentlichen rechteckförmige Grundkontur aufzuspannen. Bei der dargestellten Ausführungsform werden im Bereich der dem Übergaberad (29) und einem Eingaberad (35) zugewandten Ausdehnung der Heizstrecke (24) ein einzelnes relativ groß dimensioniertes Umlenkrad (34) und im Bereich von benachbarten umlenkungen zwei vergleichsweise kleiner dimensionierte umlenkräder (36) verwendet. Grundsätzlich sind aber auch beliebige andere Führungen denkbar.

Zur Ermöglichung einer möglichst dichten Anordnung des Übergaberades (29) und des Eingaberades (35) relativ zueinander erweist sich die dargestellte Anordnung als besonders zweckmäßig, da im Bereich der entsprechenden Ausdehnung der Heizstrecke (24) drei Umlenkräder (34, 36) positioniert sind, und zwar jeweils die kleineren Umlenkräder (36) im Bereich der Überleitung zu den linearen Verläufen der Heizstrecke (24) und das größere Umlenkrad (34) im unmittelbaren Übergabebereich zum Übergaberad (29) und zum Eingaberad (35). Alternativ zur Verwendung von kettenartigen Transportelementen (33) ist es beispielsweise auch möglich, ein rotierendes Heizrad zu verwenden.

Nach einem fertigen Blasen der Behälter (2) werden diese von einem Entnahmerad (37) aus dem Bereich der Blas-stationen (3) herausgeführt und über das Übergaberad (28) und ein Ausgaberad (38) zur Ausgabestrecke (32) transportiert.

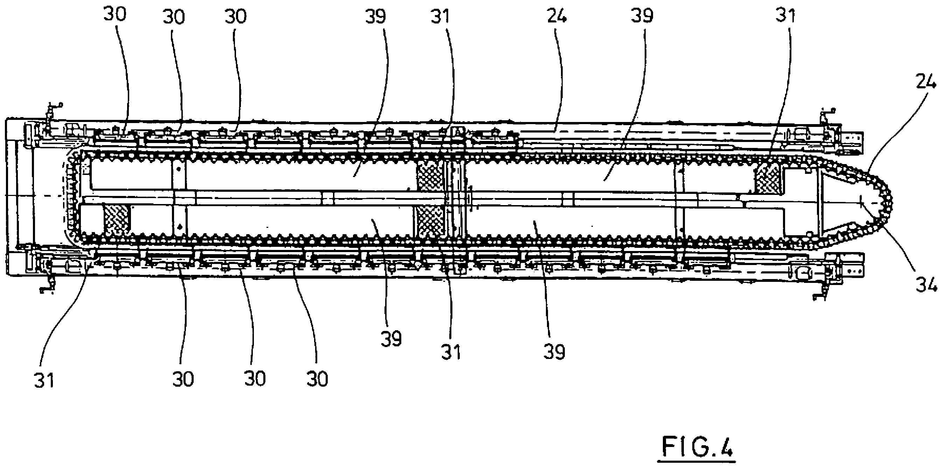

In der in Fig. 4 dargestellten modifizierten Heizstrecke (24) können durch die größere Anzahl von Heizstrahlern (30) eine größere Menge von Vorformlingen (1) je Zeiteinheit temperiert werden. Die Gebläse (31) leiten hier Kühlluft in den Bereich von Kühlluftkanälen (39) ein, die den zugeordneten Heizstrahlern (30) jeweils gegenüberliegen und über Ausströmöffnungen die Kühlluft abgeben. Durch die Anordnung der Ausströmrichtungen wird eine Strömungs-richtung für die Kühlluft im wesentlichen quer zu einer Transportrichtung der Vorformlinge (1) realisiert. Die Kühlluftkanäle (39) können im Bereich von den Heizstrahlern (30) gegenüberliegenden Oberflächen Reflektoren für die Heizstrahlung bereitstellen, ebenfalls ist es möglich, über die abgegebene Kühlluft auch eine Kühlung der Heizstrahler (30) zu realisieren.

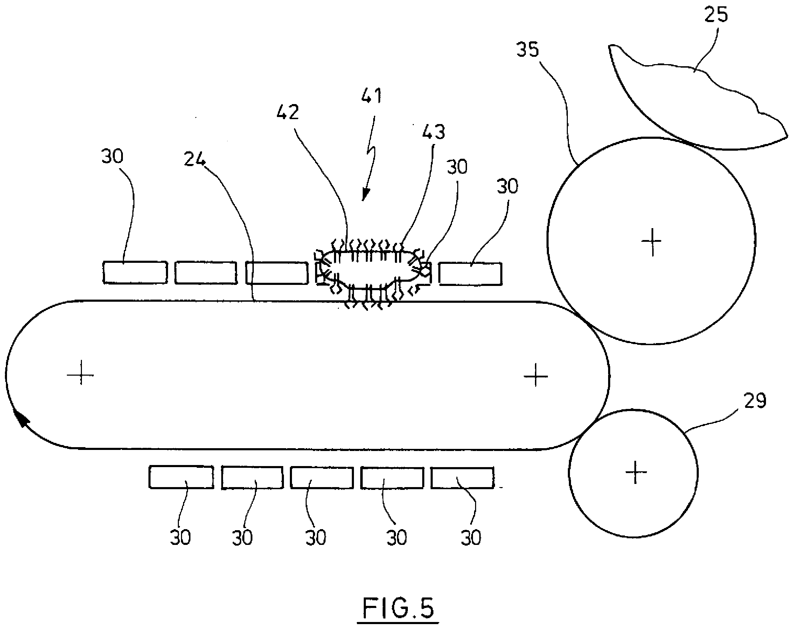

Fig. 5 zeigt schematisch und stark vereinfacht eine Anordnung ähnlich zur Darstellung in Fig. 3 mit zusätzlicher Anordnung einer Sterilisiereinrichtung (41) im Bereich der Heizstrecke (24). Gemäß dem in Fig. 5 dargestellten Ausführungsbeispiel ist im Bereich der Sterilisiereinrichtung (41) eine umlaufend angetriebene Transporteinrichtung (42) für Halteelemente (43) angeordnet, die eine Fixierung und/oder Positionierung der vorformlinge (1) mindestens während eines Teiles der Zeitdauer des Sterilisationsvorganges ermöglichen.

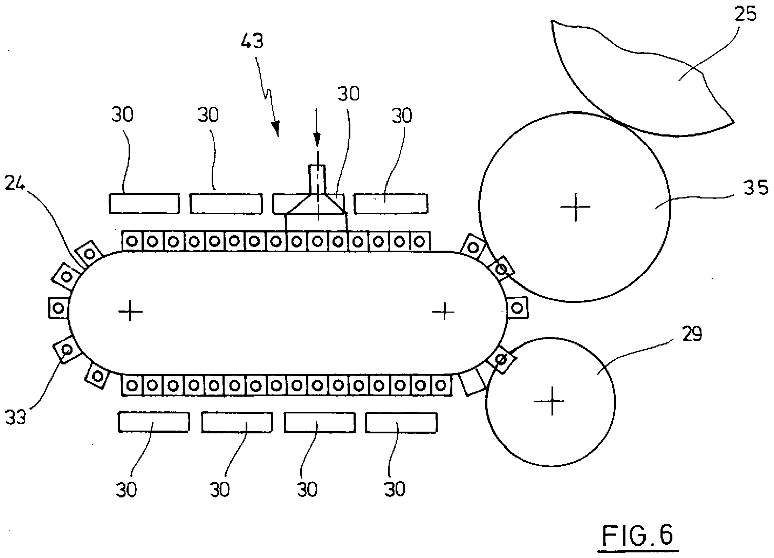

Fig. 6 zeigt eine abgewandelte Ausführungsform, bei der im Bereich der Sterilisiereinrichtung (41) keine zusätzlichen Halteelemente (42) verwendet werden. Zusätzlich eingezeichnet sind die Transportelemente (33) der Heizstrecke (24).

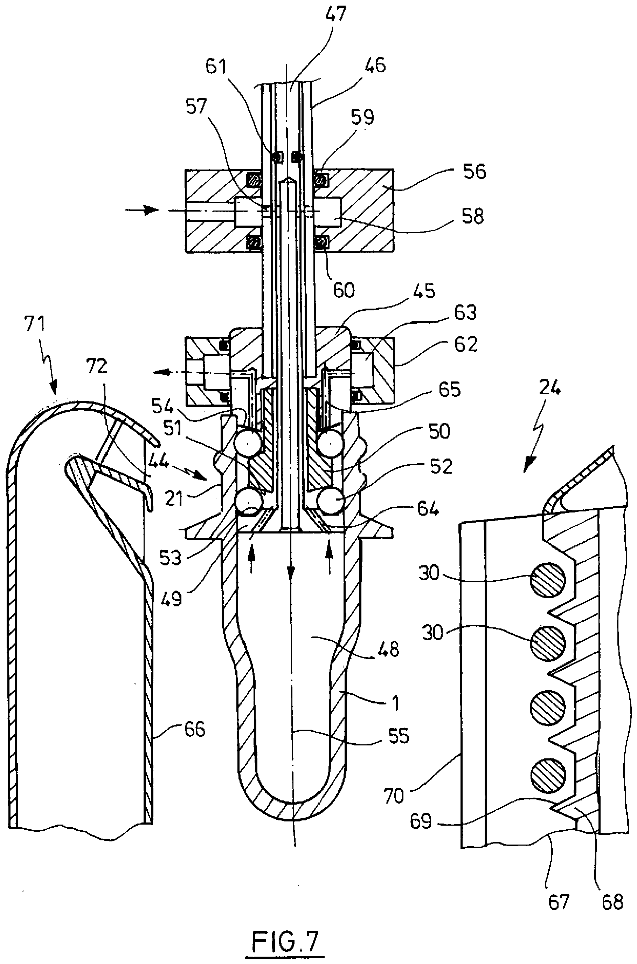

Fig. 7 zeigt einen Bereich der Sterilisiereinrichtung (41), die mit einem Applikator (44) in den Mündungsabschnitt (21) des vorformlings (1) eingeführt ist. Der Applikator (44) weist ein Basiselement (45) auf, das von einem Positionierelement (46) getragen ist. Beim dargestellten Ausführungsbeispiel ist das Positionierelement (46) rohrartig ausgebildet und durch das Positionierelement (46) erstreckt sich ein Stellelement (47) hindurch. Das Stellelement (47) mündet im Bereich seiner einem Innenraum (48) des Vorformlings (1) zugewandten Ausdehnung in ein Spannelement (49) ein.

Zwischen dem Spannelement (49) und dem Basiselement (45) ist ein Keilelement (50) angeordnet, das mindestens eine Keilfläche (51) zur Beaufschlagung mindestens eines Klemmelementes (52) aufweist. Das Klemmelement (52) ist gerundet ausgebildet. Vorzugsweise liegt eine kugelartige Ausführung des Klemmelementes (52) vor. Als Material für das Klemmelement (52) ist beispielsweise an die Verwendung einer Keramik, von PEEK oder einem mit Keramik beschichteten Metall gedacht.

Im Bereich seiner dem Keilelement (50) zugewandten Ausdehnung weist das Spannelement (49) mindestens eine Keilfläche (53) auf. Ebenfalls ist das Basiselement (45) im Bereich seiner dem Keilelement (50) zugewandten Ausdehnung mit mindestens einer Keilfläche (54) versehen.

Beim dargestellten Ausführungsbeispiel sind somit in Richtung einer Längsachse (55) des Positionierelementes (46) hintereinander und relativ zueinander mit einem Abstand mindestens zwei Klemmelemente (52) positioniert. Dies ist aber optional und nicht zwingend notwendig. Ebenfalls sind in einer Umfangsrichtung des Keilelementes (50) relativ zueinander beabstandet mindestens zwei Klemmelemente (52) angeordnet. Bevorzugt werden mindestens drei in der umfangsrichtung relativ zueinander äquidistant beabstandete Klemmelemente (52) verwendet.

Für ein Einführen des Applikators (44) in den Mündungsabschnitt (21) bzw. für ein Entfernen des Applikators (44) aus dem Bereich des Mündungsabschnittes (21) wird gegenüber der Positionierung in Fig. 7 der Abstand zwischen dem Spannelement (49) und dem Basiselement (45) durch eine Positionierung des Stellelementes (47) vergrößert. Die Klemmelemente (52) können hierdurch in Richtung auf das Stellelement (47) weiter in jeweils durch Keilflächen (51, 52) bzw. Keilflächen (51, 54) begrenzte Aufnahmetaschen hineinwandern und geben den Vorformling (1) relativ zum Applikator (44) frei. In umgekehrter Weise werden die Klemmelemente (52) bei einer Abstandsverringerung zwischen dem Spannelement (49) und dem Basiselement (45) entlang der Keilflächen (51, 53, 54) in Richtung auf den Mündungsabschnitt (21) des Vorformlings (1) positioniert und führen hierdurch einen Klemmvorgang durch.

Nach einer Positionierung des Applikators (44) im Bereich des Mündungsabschnittes (41) wird unter Verwendung einer Zuführeinrichtung (56) Sterilisationsmittel in den Bereich des Applikators (44) geleitet. Beim dargestellten Ausführungsbeispiel weist das rohrartige Positionierelement (46) mindestens eine außenseitige Ausnehmung (57) auf, die in einen Zuführraum (58) der Zuführeinrichtung (56) einmündet. Der Zuführraum (58) ist durch Dichtungen (59, 60) gegenüber einer Umgebung abgedichtet. Die Dichtungen (59, 60) können beispielsweise als O-Ringe ausgebildet sein, die das Positionierelement (46) außenseitig umgeben.

Zur Abdichtung des Stellelementes (47) relativ zum Positionierelement (46) wird eine Dichtung (61) verwendet. Auch diese Dichtung (61) kann als ein O-Ring ausgebildet sein, der in eine außenseitige nutförmige Vertiefung des Stellelementes (47) eingesetzt ist.

Zur Durchführung eines Sterilisationsvorganges wird durch die Zuführeinrichtung (56) und das Positionierelement (46) hindurch das Sterilisationsmittel in den Vorformling (1) eingeleitet. Beim dargestellten Ausführungsbeispiel ist auch das Stellelement (47) zumindest bereichsweise hohl ausgebildet, so daß die Zuführung des Sterilisationsmittels durch den hohlen Bereich des Stellelementes (47) hindurch erfolgt. Hierdurch kann eine zentrale Einleitung in den Vorformling (1) in Richtung der Längsachse (55) erfolgen.
Eine Ableitung des Sterilisationsmittels aus dem Innenraum (48) heraus kann beispielsweise durch Kanäle oder Nuten im Bereich des Applikators (44) erfolgen. Im einfachsten Fall erfolgt eine Ableitung durch geeignete Nuten an den Klemmelementen (52) vorbei in Richtung auf eine Umgebung.

Das Sterilisationsmittel wird vorzugsweise in einem gasförmigen Zustand in den Innenraum (48) eingeleitet. Insbesondere ist an eine Temperatur des Sterilisationsmittels von oberhalb 100°C gedacht. Vorzugsweise weist der Vorformling (1) bei der Durchführung des Sterilisationsvorganges im Bereich seiner zu sterilisierenden inneren Oberfläche eine Temperatur von oberhalb 80°C auf. Hinsichtlich des Sterilisationsmittels ist insbesondere an die Verwendung von Wasserstoffperoxid gedacht.

Fig. 7 veranschaulicht die Anordnung des Vorformlings (1) während der Durchführung des Sterilisationsvorganges im Bereich der Heizstrecke (24). Der Vorformling (1) wird hierbei sowohl vom Sterilisationsmittel als auch von Heizstrahlern (30) beaufschlagt. Vorzugsweise erfolgt die Beaufschlagung des Vorformlings (1) mit dem Sterilisationsmittel im Bereich der inneren Oberfläche des Vorformlings (1) und eine Beaufschlagung mit der Heizstrahlung im Bereich einer äußeren Oberfläche. Beim dargestellten Ausführungsbeispiel sind die Heizstrahler (30) entlang einer Transportrichtung der Vorformlinge (1) durch die Heizstrecke (24) einseitig angeordnet. Den Heizstrahlern (30) gegenüberliegend sind Reflektoren (66) positioniert. Typischerweise werden die Heizstrahler (30) im Bereich von Heizkästen (67) angeordnet, wobei auf einer den Vorformlingen (1) abgewandten Seite der Heizstrahler (30) vom Heizkasten (67) gehalterte Reflektoren (68) angeordnet sind. Vorzugsweise weisen die Reflektoren (68) ein Reflektionsprofil (69) auf. Zwischen den Heizstrahlern (30) und den Vorformlingen (1) kann eine Filterscheibe (70) positioniert werden, die frequenzselektive Eigenschaften aufweist. Die Filterscheibe (70) kann beispielsweise aus Quarzglas bestehen.

Vorzugsweise generieren die Heizstrahler (30) eine Heizstrahlung im NIR-Bereich. Verwendbar sind aber auch Infrarot-Strahler, lichtemittierende Dioden oder Strahleinrichtungen für Mikrowellenenergie oder HochfrequenzEnergie.

Gegebenenfalls ist auch eine Kombination von zwei oder mehr der vorstehend aufgeführten Wärmequellen möglich.

Bei einer Applikation von Mikrowellenenergie in Kombination mit einer geeigneten Abschirmung ist es möglich, gezielt eine Erhitzung von wasserhaltigem Sterilisationsmittel im Innenraum (48) des Vorformlings (1) bzw. auf dessen innerer Oberfläche vorzunehmen. Hierfür ist Wasserstoffperoxid besonders geeignet;

Gemäß dem Ausführungsbeispiel in Fig. 7 ist zur Kühlung des Mündungsabschnittes des Vorformlings (1) eine Kühleinrichtung (71) verwendet. Die Kühleinrichtung (71) weist mindestens eine Ausströmöffnung (72) für ein Kühlgas, beispielsweise gekühlte Luft, auf. Gemäß der Ausführungsform in Fig. 7 bildet die Kühleinrichtung (71) mit dem Reflektor (66) ein gemeinsames Bauteil. Beispielsweise ist es möglich, die Kühleinrichtung (71) als ein Hohlprofil auszubilden, auf dessen äußerer dem Vorformling (1) zugewendeter Oberfläche der Reflektor (66) angeordnet ist. Die Anordnung kann hierbei durch Befestigung eines Reflektorbauteiles oder durch geeignete reflektierende Beschichtungen des Hohlprofils erfolgen.

Gemäß typischen Prozeßbedingungen weist das Sterilisationsmittel während der Durchführung der Sterilisierung eine Temperatur im Bereich von 100°C bis 130°C auf. Der Vorformling (1) weist während der Durchführung der Sterilisierung mindestens im Bereich seiner inneren Oberfläche eine Temperatur von 100°C bis 130°C auf. Eine typische Sterilisationsdauer beträgt etwa 0,1 bis 0,5 sec. Als Sterilisationsmittel wird bevorzugt verdampftes Wasserstoffperoxid verwendet, das mit Heißluft gemischt wird. Die Wasserstoffperoxidkonzentration beträgt dabei etwa 15 bis 35 Gewichtsprozent.

## Patentansprüche

1. Verfahren zum Sterilisieren von Vorformlingen (1) aus einem thermoplastischen Material, die zur Herstellung von blasgeformten Behältern (2) vorgesehen sind sowie bei dem ein Sterilisationsmittel in den Bereich des Vorformlings (1) zugeführt wird, sowie bei dem die Vorformlinge (1) vor ihrer Blasverformung erwärmt werden, wobei die Erwärmung in einer Heizstrecke (24) erfolgt, in der entlang einer Transportrichtung der Vorformlinge Heizstrahler (30) einseitig angeordnet sind, **dadurch gekennzeichnet, dass** das Sterilisieren mindestens zeitweise während des Erwärmens der Vorformlinge (1) in der Heizstrecke (24) durchgeführt wird, und die Temperatur des Vorformlings (1) im Bereich einer inneren Oberfläche während der Durchführung der Sterilisierung mindestens 80°C beträgt.

2. Verfahren nach Anspruch 1, **dadurch gekennzeichnet, dass** die Temperatur in einem Bereich von 100°C bis 130°C liegt.

3. Verfahren nach einem der Ansprüche 1 oder 2, **dadurch gekennzeichnet, dass** der Vorformling (1) vor dem Sterilisieren, während des Sterilisierens sowie nach dem Sterilisieren erwärmt wird.

4. Verfahren nach einem der Ansprüche 1 bis 3 , **dadurch gekennzeichnet, dass** ein Mündungsabschnitt des Vorformlings (1) mindestens während eines Teiles des Erwärmungsvorganges gekühlt wird.

5. Verfahren nach einem der Ansprüche 1 bis 4, **dadurch gekennzeichnet, dass** mindestens während eines Teiles des Zeitraumes des Sterilisierens das Sterilisationsmittel sowohl in einen Innenraum des Vorformlings (1) eingeleitet als auch aus diesem abgeleitet wird.

6. Verfahren nach einem der Ansprüche 1 bis 5, **dadurch gekennzeichnet, dass** der Innenraum des Vorformlings (1) gegenüber einer Umgebung des Vorformlings (1) verschlossen wird.

7. Vorrichtung zum Sterilisieren von Vorformlingen (1) aus einem thermoplastischen Material, die zur Herstellung von blasgeformten Behältern vorgesehen sind, die eine Zuführeinrichtung (56) zur Beaufschlagung mindestens eines Teiles des Vorformlings (1) mit einem Sterilisationsmittel aufweist, **dadurch gekennzeichnet, dass** die Zuführeinrichtung (56) im Bereich einer Heizstrecke (24) zur Temperierung der Vorformlinge (1) angeordnet ist, wobei in der Heizstrecke (24) entlang einer Transportrichtung der Vorformlinge Heizstrahler (30) einseitig angeordnet sind, und wobei der Vorformling (1) im Bereich mindestens einer Transportposition innerhalb der Heizstrecke (24) sowohl von einem Heizelement mit Wärmeenergie als auch von der Zuführeinrichtung (56) mit dem Sterilisationsmittel beaufschlagbar ist, wobei sowohl die in Transportrichtung der Vorformlinge (1) vor der Zuführeinrichtung (56) als auch die im Bereich der Zuführeinrichtung (56) angeordneten Heizelemente dazu ausgebildet sind, den Vorformling (1) mindestens im Bereich seiner inneren Oberfläche mit einer Temperatur von mindestens 80°C zu erwärmen.

8. Vorrichtung nach Anspruch 7, **dadurch gekennzeichnet, dass** die Heizelemente eine Strahlungsleistung zur Erreichung einer Temperatur von etwa 100°C bis 130°C aufweisen.

9. Vorrichtung nach einem der Ansprüche 7 oder 8, **dadurch gekennzeichnet, dass** in einer Transportrichtung der Vorformlinge (1) sowohl vor der Zuführeinrichtung (56) als auch im Bereich der Zuführeinrichtung (56) und hinter der Zuführeinrichtung (56) Heizelemente angeordnet sind.

10. Vorrichtung nach einem der Ansprüche 7 bis 9, **dadurch gekennzeichnet, dass** im Bereich der Heizstrecke (24) mindestens eine Kühleinrichtung (71) zur Kühlung von Mündungsabschnitten der Vorformlinge (1) angeordnet ist.

11. Vorrichtung nach einem der Ansprüche 7 bis 10, dass die Zuführeinrichtung (56) sowohl Mittel zur Zuführung als auch Mittel zur Ableitung des Sterilisationsmittels aufweist.

12. Vorrichtung nach einem der Ansprüche 7 bis 11, **dadurch gekennzeichnet, dass** im Bereich der Heizstrecke (24) mindestens ein Verschlusselement angeordnet ist, das einen Innenraum des Vorformlings (1) gegenüber einer Umgebung verschließt.

## Claims

1. A method for sterilising preforms (1) made of thermoplastic material, which are provided for producing blow-moulded containers (2), wherein a sterilizing means is supplied into the vicinity of the preform (1), wherein the preforms (1) are heated before being blow-moulded, wherein the heating is performed in a heating section (24) in which radiant heaters (30) are arranged on one side along a transport direction of the preforms
**characterised in that**
- the sterilisation is performed in the heating section (24) at least temporarily while the preforms (1) are being heated and that the temperature of the preform (1) in the vicinity of an inner surface is at least 80 °C during the sterilisation procedure.

2. The method in accordance with Claim 1, **characterised in that** the temperature ranges from 100 °C to 130 °C.

3. The method in accordance with any one of Claims 1 or 2, **characterised in that** the preform (1) is heated prior to sterilisation, during sterilisation as well as subsequent to sterilisation.

4. The method in accordance with any one of Claims 1 to 3, **characterised in that** an outlet section of the preform (1) is cooled at least during a part of the heating procedure.

5. The method in accordance with any one of Claims 1 to 4, **characterised in that**, at least during a part of the sterilisation period, the sterilisation means is introduced into and discharged from an interior region of the preform (1).

6. The method in accordance with any one of Claims 1 to 5, **characterised in that** the interior region of the preform (1) is closed against an environment of the preform (1).

7. A device for sterilising preforms (1) made of thermoplastic material, which are provided for producing blow-moulded containers, the device having a supply apparatus (56) for applying a sterilisation means to at least a part of the preform (1), **characterised in that** the supply apparatus (56) is arranged in the vicinity of a heating section (24) in order to control the temperature of the preforms (1), wherein radiant heaters (30) are arranged on one side along a transport direction of the preforms and wherein, in the vicinity of at least one transport position within the heating section (24), the sterilisation means is applied to the preform (1) both by a heating element having thermal energy and by the supply apparatus (56), wherein the heating elements arranged upstream of the feeding apparatus (56) in the transport direction of the preforms (1) as well as the heating elements arranged in the vicinity of the supply apparatus (56) are configured to heat the preform (1) to a temperature of at least 80 °C at least in the vicinity of its inner surface.

8. The device in accordance with Claim 7, **characterised in that** the heating elements have a radiation performance to achieve a temperature of about 100 °C to 130 °C.

9. The device in accordance with any one of Claims 7 or 8, **characterised in that**, in a transport direction of the preforms (1), heating elements are arranged both upstream of the feeding apparatus (56) and in the vicinity of the feeding apparatus (56) as well as downstream of the feeding apparatus (56).

10. The device in accordance with any one of Claims 7 to 9, **characterised in that** at least one cooling apparatus (71) is arranged in the vicinity of the heating section (24) in order to cool outlet sections of the preforms (1).

11. The device in accordance with any one of Claims 7 to 10, **characterised in that** the feeding apparatus (56) has means to supply the sterilisation means as well as means to discharge the sterilisation means.

12. The device in accordance with any one of Claims 7 to 11, **characterised in that** at least one closing element is arranged in the vicinity of the heating section (24), the closing element closing an interior region of the preform (1) against an environment.

## Revendications

1. Procédé de stérilisation de préformes (1) en un matériau thermoplastique destinées à la fabrication de récipients (2) moulés par soufflage, dans le cadre duquel un agent de stérilisation est introduit dans la zone de la préforme (1), et dans le cadre duquel les préformes (1) sont réchauffées avant leur moulage par soufflage, le réchauffement ayant lieu dans une section de chauffage (24) dans laquelle des radiateurs de chauffage (30) sont agencés unilatéralement le long d'une direction de transport des préformes **caractérisé en ce que** la stérilisation a, temporairement au moins, lieu pendant le réchauffement des préformes (1) dans la section de chauffage (24) et que la température de la préforme (1) au niveau d'une surface intérieure pendant la réalisation de la stérilisation est d'au moins 80 °C.

2. Procédé selon la revendication 1, **caractérisé en ce que** la température est comprise dans un intervalle de 100 °C à 130 °C.

3. Procédé selon l'une des revendications 1 ou 2, **caractérisé en ce que** la préforme (1) est réchauffée avant la stérilisation, pendant la stérilisation et après la stérilisation.

4. Procédé selon l'une des revendications 1 à 3, **caractérisé en ce qu'**une section d'embouchure de la préforme (1) est refroidies pendant au moins une partie du processus de réchauffement.

5. Procédé selon l'une des revendications 1 à 4, **caractérisé en ce que** l'agent de stérilisation est, pendant au moins une partie de l'intervalle de temps de stérilisation, non seulement introduit dans un espace intérieur de la préforme (1), mais aussi évacué de celui-ci.

6. Procédé selon l'une des revendications 1 à 5, **caractérisé en ce que** l'espace intérieur de la préforme (1) est obturé par rapport à un milieu qui entoure la préforme (1).

7. Dispositif de stérilisation de préformes (1) en un matériau thermoplastique destinées à la fabrication de récipients moulés par soufflage présentant un dispositif d'adduction (56) d'un agent de stérilisation agissant sur une partie au moins de la préforme (1) **caractérisé en ce que** le dispositif d'adduction (56) est agencé dans la zone d'une section de chauffage (24) dans laquelle sont tempérées les préformes (1), des radiateurs de chauffage (30) étant agencés unilatéralement le long d'une direction de transport des préformes dans la section de chauffage (24) et la préforme (1) pouvant, dans la zone d'au moins une position de transport à l'intérieur de la section de chauffage (24), être soumise non seulement à l'énergie thermique d'un élément de chauffage, mais aussi à l'agent de stérilisation éjecté par le dispositif d'adduction (56), les éléments de chauffage agencés aussi bien avant le dispositif d'adduction (56) dans le sens de transport des préformes (1) que ceux agencés dans la zone du dispositif d'adduction (56) étant conçus pour réchauffer la préforme (1), au moins au niveau de sa surface intérieure, à une température d'au moins 80 °C.

8. Dispositif selon la revendication 7, **caractérisé en ce que** les éléments de chauffage ont une puissance de rayonnement permettant d'atteindre une température d'environ 100 °C à 130 °C.

9. Dispositif selon l'une des revendications 7 ou 8, **caractérisé en ce que** des éléments de chauffage sont, dans le sens de transport des préformes (1), agencés non seulement avant le dispositif d'adduction (56), mais aussi dans la zone du dispositif d'adduction (56) et derrière le dispositif d'adduction (56).

10. Dispositif selon l'une des revendications 7 à 9, **caractérisé en ce qu'**au moins un dispositif de refroidissement (71) pour le refroidissement de sections d'embouchure des préformes (1) est agencé au niveau de la section de chauffage (24).

11. Dispositif selon l'une des revendications 7 à 10, **caractérisé en ce que** le dispositif d'amenée (56) présente non seulement des moyens d'adduction mais aussi des moyens d'conduit d'évacuation de l'agent de stérilisation.

12. Dispositif selon l'une des revendications 7 à 11, **caractérisé en ce qu'**au moins un élément d'obturation est agencé au niveau de la section de chauffage (24), lequel élément obture un espace intérieur de la préforme (1) par rapport à un milieu qui l'entoure.
